# EUROPEAN PATENT SPECIFICATION

(11) **EP 4 430 957 B1**
(45) Date of publication and mention of the grant of the patent: **19.08.2026**
(21) Application number: 24163999.6
(22) Date of filing: 15.03.2024
(51) Int. Cl.: A23K 50/10, A23N 17/00, G06Q 50/02, A23K 50/00, G01N 33/02

(54) **PROCESS FOR CONTROLLING A CATTLE BREEDING FACILITY**
VERFAHREN ZUR STEUERUNG EINER VIEHZUCHTANLAGE
PROCÉDÉ DE COMMANDE D'UNE INSTALLATION D'ÉLEVAGE DE BÉTAIL

(30) Priority: 17.03.2023 IT 202300005058
(43) Date of publication of application: 18.09.2024
(73) Proprietor: Faresin Industries S.p.A., 36042 Breganze VI (IT)
(72) Inventor: ANDRIGHETTO, Igino, 36100 Vicenza (VI) (IT); SERVA, Lorenzo, 35030 Rubano (PD) (IT); BISON, Giacomo, 35010 Limena (PD) (IT); MAGRIN, Luisa, 35010 Villa del Conte (PD) (IT); MARCHESINI, Giorgio, 36100 Vicenza (VI) (IT); FARESIN, Sante, 36042 Breganze (VI) (IT); FARESIN, Silvia, 36042 Breganze (VI) (IT); FARESIN, Giulia, 36042 Breganze (VI) (IT)
(74) Representative: Caldon, Giuliano

(56) References cited:
- EP-A1- 4 118 957
- US-A- 5 668 718
- US-A1- 2004 199 275
- US-A1- 2006 185 605
- US-A1- 2008 189 085
- US-A1- 2015 245 595
- US-A1- 2016 324 188
- "Herd Health and Production Management in Dairy Practice", 1 January 2001, WAGENINGEN PERS, article ANDY L. SKIDMORE ET AL: "Monitoring milk production: defining preset targets and execution", pages: 223 - 281, XP055292499

## Description

### Field of application

The present invention refers to a process for controlling a cattle breeding facility according to the preamble of the independent claim.

The present process is inserted in the field of zootechny and is advantageously intended to be employed in the livestock field, for example by stall veterinarians, diet or formula specialists, in intensive cattle breeding, in particular for producing milk or meat.

In particular, the process for controlling a breeding facility, object of the present invention, is particularly recommended for determining, analyzing and controlling the diet and wellbeing conditions of the cattle.

In addition, the process for controlling a breeding facility, object of the present invention, is advantageously intended to be used for modifying such conditions in order to optimize the production of milk or meat with respect to the quantity of food distributed to the animals.

### State of the art

The intensive (or industrial) breeding, in particular of cattle, generally occurs in stalls that are normally provided with one or more corridors, along which the mangers of the animals are placed.

In the stables/stalls of intensive (or industrial) animal farms, the animals are generally fed with a food product (in the j argon of the field termed unifeed or TMR (Total Mixed Ratio)) constituted by a mixture of foods, such as forages (like corn, sorghum, etc.) and concentrated foods (such as soy grain, corn grain, etc.). The unifeed is therefore a one-course meal including the correct equilibrium between fibrous foods (which stimulate rumination by facilitating a digestive-metabolic process that supports the production of milk and the content of fat in the milk), farm energy foods, feed and integrators.

From a chemical or nutritional standpoint, the ration is generally constituted for example by about 50% water, 5% ash, 8% protein, 3% fat, 19% fiber and 15% start. Each food component brings - each with is different ratio - all the nutritional factors that delineate the total chemical composition of the unifeed. Historically and for practical reasons, the chemical or nutritional composition of the unifeed can be expressed as a percentage with respect to the total quantity moist food (as is), or as a percentage with respect to the total quantity of food lacking water, i.e. with respect to the quantity of dry matter of the food. In both cases, this is a chemical or nutritional composition respectively expressed as "% as is" or "%ss" [ss = sostanza secca = dry matter]. The water contained in the ration is commonly indicated as "moisture" of the ration.

The unifeed ration is generally formulated by the dietary specialist who bases his/her own choices by considering several characteristics of the herd (breed, mean production, lactation phase, number of lactations, age of the animals, ponderal growth etc.), the farm and extra-farm availability of the single food components, the supply costs, etc. The food needs of the bred animals (for the production of milk or meat) differ as a function of many factors, such as the type of production, the age of the animal, the physical condition of the animal, the level and the phase of production (lactation or fattening phase), etc. Therefore, in common practice, the animals are grouped into uniform groups for several of the above-described factors. It follows that, within a livestock farm, various groups of animals coexist that are fed in a manner consistent with their own metabolic and production needs. In the literature and in practice, there are several guidelines that assist the dietary specialist in the formulation of the unifeed diets, such to maximize the production, decrease waste and preserve animal wellbeing, through the balanced supply of the nutritional elements.

The cost of the feeding in the dairy and beef cattle operation generally affects between 50-80% of the total production cost. Known in the field therefore is the need to improve the efficiency of transformation of the ingested foods into productions of milk and meat in order to improve company profit.

It is also known that the farm efficiency essentially depends on two types of factors, including one food and one management.

With regard to the food aspects, the guidelines present in the literature provide for suitably combining, in a qualitative and quantitative manner, the nutritional factors administered for enhancing the digestive capacities of the animals in order to substantially reduce the production costs. The improvement of the digestive use of the rations, in addition to having positive effects on the costs of the production system, decreases the environmental impact, rendering the productions more sustainable, since it reduces the excretions with consequent lower dispersions of waste on the territory and contains the methane gases emitted per unit of product (kg of milk or kg of meat).

An example of the aforesaid guidelines is given by Skidmore et al. "Monitoring milk production: defining preset targets and execution." Herd Health & Production Management in Dairy Practice (1996). Such document defines objectives for a herd health and production management programme for milk production and describes how such a programme can be executed, in particular how to increase milk production over time with good practice.

The processes for controlling breeding facilities of known type, based on the aforesaid guidelines, have in practice shown that they do not lack drawbacks.

The main drawback of such process lies in the fact that the improvement of the digestibility does not only depend on administered nutritional factors, but also on the modes of preparation and administration of the rations and in particular for the TMR diets (Total Mixed Ration) on their chemical-physical homogeneity and on the capacity of selection, by the animals, of the foods at the time of consumption. Therefore, the existing guidelines are not entirely complete, since they do not consider important factors that affect the production efficiency.

A further drawback of the process of known type lies in the fact that in order to optimize the production of meat or milk, in all cases only factors tied to the composition and to the administered quantity of food are analyzed. Causes different from and independent of those of the diet are instead not identified, né nor is the impact of such causes on the production efficiency quantified. Otherwise, in addition to factors tied to feeding, also management affects the transformation efficiency, since animals bred in conditions of poor wellbeing, lacking control of the cattle, can negatively affect the productivity.

A further drawback of the process of known type lies in the fact that presently the diet efficiency is expressed in terms of kg of milk or meat produced daily for 1 kg of dry matter of TMR ingested. Nevertheless, this indicator is unable to discern which are the possible limiting effects linked to the diet and which are linked to the general management.

An alternative process is also known from document US 2006185605, which discloses a method for controlling a livestock breeding facility via a centralized control and data collection and transfer system. The centralized system creates food analysis reports which are used in the analysis of animal performance and the effectiveness of the food delivery process. Furthermore, production data (indicative of the actual meat production of the cattle) is collected, recorded and in turn used to analyze individual animals or groups of animals for return on investment and producer evaluation. All the data collected in the system is processed to obtain, through mathematical relationships, recommended actions for cattle breeding.

### Presentation of the invention

The main object of the present invention is therefore to overcome the drawbacks manifested by the solutions of known type, by providing a process for controlling a cattle breeding facility, which allows increasing the production efficiency of the breeding facility.

Another object of the present invention is to provide a process for controlling a cattle breeding facility, which allows optimizing the production of milk or meat of the breeding facility.

Another object of the present invention is to provide a process for controlling a cattle breeding facility, which allows determining and distributing, to the cattle, an optimal and balanced quantity of food.

Another object of the present invention is to provide a process for controlling a cattle breeding facility, which allows remedying problems of the breeding facility that are not tied to food aspects.

Another object of the present invention is to provide a process for controlling a cattle breeding facility that is simple and inexpensive to implement.

The invention is defined by the subject-matter of the claims.

### Brief description of the drawings

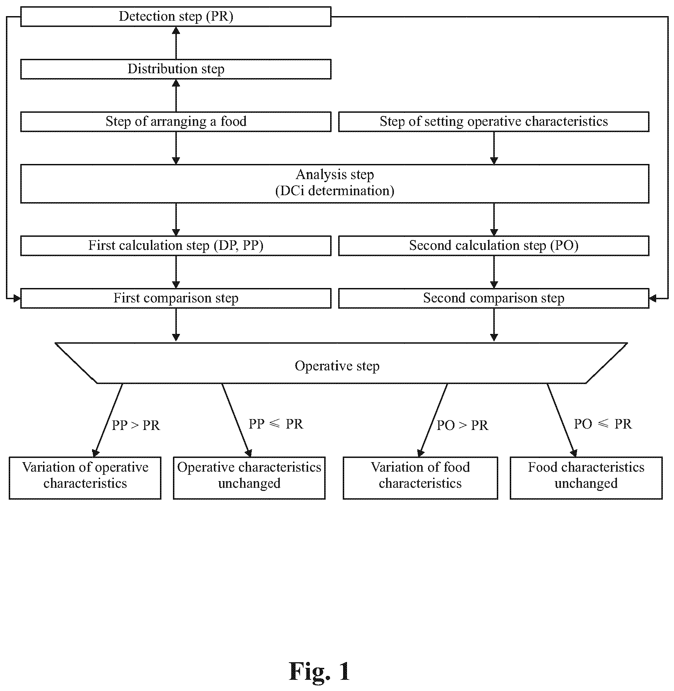

The technical characteristics of the invention, according to the aforesaid objects, are clearly seen in the contents of the below-reported claims and the advantages thereof will be more evident from the following detailed description, made with reference to figure 1 which represents a flow diagram relative to a merely exemplifying and non-limiting embodiment of the process for controlling a cattle breeding facility, object of the present invention.

### Detailed description of a preferred embodiment

The present process for controlling a cattle breeding facility is intended to be advantageously employed in intensive breeding operations in order to determine, analyze and control the diet and wellbeing conditions of the livestock animals, such as cattle. In addition, the process is intended to be advantageously employed for modifying such conditions in order to optimize the production of milk or meat with respect to the quantity of food distributed.

Hereinbelow, the expressions "breeding facility", "livestock breeding" and "livestock farm" can be used alternatively with the same significance.

The process for controlling a cattle breeding facility according to the invention comprises a step of arranging a food, in which at least one preset quantity of food is prepared for a dairy or beef cattle breeding facility.

More in detail, the food intended to be distributed to the animals (termed unifeed or Total Mixed Ratio (TMR) in the jargon of the field) usually comprises a mixture of solid bodies (in the form of ground plants and/or granules) of multiple foods. In particular, the mixture of the food product comprises forages (such as corn, sorghum, etc.) and concentrated foods which for example contain protein components (such as soy grain) and/or energy components (such as corn grain). Advantageously, the food is arranged in accordance with at least one food recipe, which comprises different quantities of the aforesaid food components. Advantageously, the food can be arranged in a silos, in one or more storage bales, in a collection conveyor, in a containment box of a cutting and/or processing machine, for example a mill or a mixer wagon.

Of course, without departing from the protective scope of the present invention, in the arranging step, additional pre-established quantities of food (equal or different) can be arranged. In such case, the process can be executed by considering each quantity of food, or a mixture of the pre-established quantities of food, or an equivalent quantity of food, which is representative of an overall mean of the different quantities of food considered.

The process according to the invention comprises, in addition, a setting step, in which at least one operative characteristic of the breeding facility, in which the cattle are bred, is set.

With the expression "operative characteristic" it will be intended any characteristic of the breeding facility which is not directly tied to or depending on the composition of the food and on the quantity and frequency of administration of the latter. Advantageously, such operative characteristic is a factor that affects the physical and psychological wellbeing of the animal.

For example, the operative characteristic is selected from among: number of places occupied in manger, extension of the manger or drinking trough, number of places occupied in a bunk, surface area available for each bovine, presence of ventilation, presence of enzymes in litter, sewage removal frequency, frequency of the veterinary activities, space available in the manger and/or in the stall for each bovine.

The process comprises, in addition, an analysis step, in which the food is analyzed in order to obtain at least one composition data DCi, comprising at least one percentage of dry matter SS% and/or a percentage of at least one nutritional factor in the food.

More in detail, the nutritional factor of the composition data DCi is selected from among: raw protein (PG) contained in the food, raw lipids (LG) contained in the food, fibrous fraction (FF) contained in the food and reserve carbohydrates (CR) contained in the food.

Therefore, the at least one composition data DCi advantageously comprises at least one from among percentage of dry matter SS%, percentage of raw protein PG%, percentage of raw lipids LG%, percentage of fibrous fraction FF% and percentage of reserve carbohydrates CR% contained in the food.

Advantageously, the fibrous fraction (FF) is at least one selected from among: alpha-amylase-treated neutral detergent resistant fiber (aNDF), acidic detergent resistant fiber (ADF), acidic detergent resistant lignin (ADL), cellulose, hemicellulose and raw fiber.

Advantageously, in addition, the reserve carbohydrates are at least one selected from among: starch, sugars, non-structural carbohydrates (NSC) and nitrogen-free extracts.

Advantageously, the composition data points DCi in the analysis step are obtained with methods of type known in the field of chemical-physical analysis of the foods and in the field of zootechnics.

For example, the alpha-amylase-treated neutral detergent fiber fraction (aNDF) can be calculated in particular with the Van Soest method (Van Soest P.J. 1967. Development of a Comprehensive System of Feed Analyses and its Application to Forages. Journal of Animal Science. 26(1):119-128. doi:10.2527/jas1967.261119x; Van Soest P.J., Robertson J.B., Lewis B.A. 1991. Methods for Dietary Fiber, Neutral Detergent Fiber, and Nonstarch Polysaccharides in Relation to Animal Nutrition. Journal of Dairy Science. 74(10):3583-3597. doi:10.3168/jds.S0022-0302(91)78551-2).

Advantageously, the composition data DCi also comprises at least one physical characteristic CF of the food. Such physical characteristic CF is selected for example from among the length of the fiber (LMG), physically effective fiber (peNDF) and physically effective factor (pef).

In particular, the physically effective fiber fraction (peNDF) is calculated by means of the product between alpha-amylase-treated neutral detergent fiber fraction (aNDF), for example obtained as described above, and physical efficiency factor (pef).

Advantageously, in the analysis step the physical efficiency factor (pef) can be estimated by means of the calculation of the fibrous fraction (dry matter) retained by meshes of a sieve with diameter of the holes of 1.18 mm. For example, in the analysis step, a sieve can be used of type equivalent or similar to the Penn State Particle Separator (Kononoff P.J., Heinrichs A.J., Buckmaster D.R. 2003. Modification of the Penn State Forage and Total Mixed Ration Particle Separator and the Effects of Moisture Content on its Measurements. Journal of Dairy Science. 86(5):1858-1863. doi:10.3168/jds.S0022-0302(03)73773-4) and/or tabulated values of pef (Mertens D.R. 1997. Creating a System for Meeting the Fiber Requirements of Dairy Cows. Journal of Dairy Science. 80(7):1463-1481. doi:10.3168/jds.S0022-0302(97)76075-2).

As an alternative to or in combination with the above-indicated methods, the analysis step, for obtaining the composition data DCi, can be fully or partly executed by means of a detection device arranged for directly detecting one or more of the previously-reported composition data points DCi. Advantageously, the detection device comprises a NIR spectrometer operating in the near infrared spectrum and suitably configured. In particular, the composition data DCi can be calculated with any forecast method and/or with the aid of specific equations resulting from the use of the NIR spectrometer and from the analyses of images acquired with such detection device.

More in detail, the NIR spectrometer operates in the near infrared spectrum and comprises, in a manner per se known to the man skilled in the art of the field, , at least one transmitter arranged for emitting at least one beam of radiations, in particular with spectral band 900-1700 nm, intended to hit the analyzed dose of cut forage (or a sample of cut forage), and an optical receiver arranged for detecting the spectrum of a reflected radiation beam coming from the dose of cut forage hit by the aforesaid radiation beam. The spectrometer also comprises an operational unit, preferably provided with an electronic processor, arranged for receiving the measurement of the spectrum of the reflected beam, detected by the optical receiver and for calculating the aforesaid control parameter DCi. Advantageously, the analysis step is executed on a food sample after the step of arranging the food itself, e.g. after sampling a previously-arranged fraction of the latter.

Of course, without departing from the protective scope of the present invention, the analysis step can be executed during the step of arranging the food, e.g. during the loading or the mixing of the food in a mixer wagon. Alternatively or in combination, the analysis step can be executed before the step of arranging the food, for example by detecting composition data points DCi of the single food components of the latter and estimating, in an approximate manner, the composition data points DCi of the food based on the percentage of the single food components in the preset quantity of food.

Advantageously, the process also comprises a step of sampling the food, preferably provided before the analysis step, in which at least one plurality of food samples is identified and/or picked up. In particular, the analysis step is executed on each food sample of the plurality of food samples.

The present process also comprises a distribution step, in which the preset quantity of food is distributed along a manger to a plurality of cattle of the breeding facility. Advantageously, the distribution step is executed after the analysis step. Of course, without departing from the protective scope of the present invention, the distribution step can be executed before or simultaneously with respect to the analysis step. In such case, the analysis step can be executed directly on the food in the manger (e.g. by picking up and analyzing a sample from the food distributed in the manger) or during the distribution on a mixer wagon (e.g. by means of a NIR spectrophotometer mounted on the latter).

In accordance with the idea underlying the present invention, the process comprises a first calculation step, preferably implemented by means of at least one mathematical model (described more in detail below), in which a potential digestibility parameter DP and a potential production parameter PP are calculated. The potential digestibility parameter DP is indicative of the digestibility of the food and is calculated, preferably by means of the aforesaid mathematical model, as a function of at least one aforesaid composition data DCi. More in detail, the potential digestibility parameter DP is substantially indicative of the digestibility of the dry matter present in the food.

Advantageously, the potential digestibility parameter DP is measured in percentage.

In addition, the potential production parameter PP is indicative of an estimated quantity of milk or meat production obtainable from the cattle, and is calculated, preferably by means of the aforesaid mathematical model, as a function of the potential digestibility parameter DP.

Advantageously, in addition, the potential production parameter PP is measured in kilograms of milk or meat (potentially) produced in one day by a bovine.

The process according to the invention also comprises a detection step, in which an actual production parameter PR is detected that is indicative of the actual milk or meat production of the cattle.

Advantageously, the actual production parameter PR is measured in kilograms of milk or meat produced in a day by a bovine.

The process according to the invention also comprises a first comparison step, in which the comparison is executed between the potential production parameter PP and the actual production parameter PR.

In particular, in the comparison step, the values of the potential production parameter PP and of the actual production parameter PR are compared in order to determine which is the greatest of these.

The process also comprises an operative step, in which if the potential production parameter PP is greater than the actual production parameter PR, at least one operative characteristic is modified. Otherwise, if the potential production parameter PP is smaller than or equal to the actual production parameter PR, the operative characteristic is maintained unchanged.

In this manner, it is possible to determine if the set operative characteristics affect or do not affect the actual production PR of the breeding facility and if necessary modify them without necessarily acting on the quantity or the composition of the distributed food. Therefore, the process allows controlling and, if necessary, varying one or more of the operative characteristics independent of the cattle feed. Of course, without departing from the protective scope of the invention, the first comparison step can be executed in other equivalent modes which allow understanding which is the greater production parameter in order to possibly modify the operative characteristics in the operative step. For example, in accordance with an embodiment variant, in the first comparison step, a first difference is calculated between the potential production parameter PP and the actual production parameter PR. Therefore, in accordance with such embodiment variant, in the operative step the operative characteristic is modified if the first difference is greater than zero, while it is maintained unchanged if the first difference is smaller than or equal to zero.

In accordance with the present invention, the potential digestibility parameter DP is calculated with the following formula (I): $DP = {β}_{0} + {\sum }_{n} \left(\pm {β}_{i}\times DCi\right)$ in which:
DCi is the value of a composition data, and
β₀ and βᵢ are first coefficients obtained by means of statistical regression.

For example, one or more composition data points DCi previously described in the formula (I) can be substituted. Therefore, in such case in the formula (I), the equation (I') holds true: $\begin{matrix}{\sum }_{n} \left(\pm {β}_{i}\times DCi\right) = {β}_{1} \times SS % \pm {β}_{2} \times PG % \pm {β}_{3} \times LG % \pm {β}_{4} \times FF % \pm \\ {β}_{5} \times CR % \pm {β}_{6} \times CF\end{matrix}$ in which:
DCi is the value of one said composition data,
SS% is the percentage of dry matter contained in the food,
PG% is the percentage of raw protein contained in the food,
LG% is the percentage of raw lipids contained in the food
FF% is the percentage of fibrous fraction contained in the food,
CR% is the percentage of reserve carbohydrates contained in the food,
CF is a physical characteristic of the food, and
β₀, βᵢ, β₁, β₂, β₃, β₄, β₅ and β₆ are first coefficients obtained by means of statistical regression. In particular, the first coefficients are obtained by means of multivariate regression based on data obtained from a plurality of livestock farms. Advantageously, such first coefficients are contained in the mathematical model that implements the first calculation step.

By substituting the equation (I') in the formula (I), one then obtains the formula (I") reported hereinbelow: $\begin{matrix}DP = {β}_{0} + {β}_{1} \times SS % \pm {β}_{2} \times PG % \pm {β}_{3} \times LG % \pm {β}_{4} \times FF % \pm {β}_{5} \times CR % \pm \\ {β}_{6} \times CF\end{matrix}$

In accordance with an embodiment variant of the process, as an alternative to the above-reported formula (I), the potential digestibility parameter DP can be calculated based on nutritional factors detected in the food before ingestion and in the feces of the cattle after the digestion of the food.

In such case, in the analysis step, an initial composition data DCi0 is advantageously detected in the food, indicative of a percentage of a nutritional factor in the preset quantity of food before ingestion.

In addition, in the analysis step, a final composition data DCif is advantageously detected, indicative of a percentage of the nutritional factor contained in the feces of the cattle fed with said preset quantity of food. Such final composition data DCif is for example detected in a per se known manner by means of chemical analysis on the feces of the animals.

Advantageously, it is possible to calculate the potential digestibility parameter of a single nutritional factor DPi, in particular by means of the formula (IA) reported hereinbelow: $DPi = \left\{1-\left[\left(DCif/Lf\right)/\left(DCi0/L0\right)\right]\right\} \times 100$ in which:
DPi is the potential digestibility parameter of the single nutritional factor expressed in percentage (%),
DCif is the value of the final composition data, i.e. of the excreted nutritional factor, in particular present in the feces, expressed in percentage with respect to the dry matter (%ss),
DCi0 is the value of the initial composition data, i.e. of the ingested nutritional factor, in particular present in the food, expressed in percentage with respect to the dry matter (%ss),
Lf is the excreted lignin, in particular present in the feces, expressed in percentage with respect to the dry matter (%ss), and
L0 is the ingested lignin, in particular present in the food, expressed in percentage with respect to the dry matter (%ss).

In addition, it is possible to calculate the overall potential digestibility parameter DP of the dry matter, in particular by means of the formula (IB) reported hereinbelow: $DP = \left\{1-\left[\left(100/Lf\right)/\left(100/L0\right)\right]\right\} \times 100$

By simplifying the formula (IB), one obtains the formula (IC) reported hereinbelow: $DP = \left\{1-\left[L0/Lf\right]\right\} \times 100$

The latter embodiment variant is more accurate, since it gives values of the potential digestibility parameter DP that substantially coincide with the actual digestibility of the food, but it is more complex since it requires analysis directed on many samples, not only of food but also of animal feces. In addition, such embodiment variant in particular requires constantly or frequently monitoring the cattle in order to allow correlating the initial composition data points DCi0 of the ingested food with the final composition data points DCif in the feces of the animals.

In accordance with the present invention, the potential production parameter PP is obtained by means of the following formula (II): $PP = {γ}_{0} + {γ}_{1} \times SSPD + {γ}_{2} \times IO$ in which:
IO is a food homogeneity index,
*γ*₀, *γ*₁ and *γ*₂ are second coefficients obtained by means of statistical regression,
and SSPD is a potential quantity of dry matter ingested and digested by a bovine.

The potential quantity of dry matter ingested and digested by a bovine SSPD is calculated by means of the following formula (III): $SSPD = QSS \times DP$ in which:
QSS is a quantity of ingested dry matter and
DP is the potential digestibility parameter.

In particular, the second coefficients are obtained by means of multivariate regression based on data obtained from a plurality of livestock farms. Advantageously, such second coefficients are contained in the mathematical model which implements the first calculation step.

Advantageously, in addition, the aforesaid homogeneity index IO is calculated with methods of known type, e.g. by means of the method described in the patent IT 102016000047355. In such case, the homogeneity index is advantageously measured as a value comprised between 0 and 100.

Advantageously, in addition, the quantity of ingested dry matter QSS is calculated by multiplying the preset quantity of food (distributed and ingested by the) by the percentage of dry matter SS%.

In accordance with the preferred embodiment of the present invention, the process also comprises a second calculation step, preferably implemented by means of the mathematical model indicated above or a different mathematical model, in which an objective production parameter PO is calculated that is indicative of an objective production of milk or meat.

In particular, the second calculation step is executed simultaneously with the first calculation step.

Of course, without departing from the protective scope of the invention, the second calculation step can also occur before or after the first calculation step. Advantageously, the objective production parameter PO is measured in kilograms of milk or meat produced in a day by a bovine.

Advantageously, the process also comprises a second comparison step, in which the comparison is executed between the objective production parameter PO and the actual production parameter PR.

In particular, the second comparison step is executed simultaneously with the first comparison step.

Of course, without departing from the protective scope of the invention, the second comparison step can also occur before or after the first comparison step.

Advantageously, in addition, in the operative step, if the objective production parameter PO is greater than the actual production parameter PR, at least one characteristic of the food is modified. Otherwise, if the objective production parameter PO is smaller than or equal to the actual production parameter PR, the characteristic of the food is maintained unchanged.

Advantageously, the characteristic of the food is selected from among: preset quantity of food, homogeneity of the food, selectionability of the food, formulation of the food recipe, frequency with which the distribution step is executed.

For example, the homogeneity of the food is determined as "homogeneity index", in particular by means of the method described in the patent IT 102016000047355 as indicated above, and can be modified by varying the food mixing times.

In addition, the selectionability of the food is advantageously determined as "selective capacity numerical index", in particular by means of a known method described in the patent IT 102017000011157, and can be modified by varying the dimensions and/or the cutting lengths of the food components.

In this manner, it is possible to determine if the set characteristics of the food, e.g. quantity or composition, affect or do not affect the actual production PR of the breeding facility and if it is necessary to modify them without necessarily acting on the operative characteristics. Therefore, the process allows controlling, and, if necessary, varying one or more of the characteristics of the food independent of the management of the cattle wellbeing.

Therefore, the process allows distinguishing the cases in which the lack of attainment of the maximum productivity is due to operative characteristics from the cases in which it is due to characteristics of the food. Advantageously, therefore, the process allows controlling the breeding facility in a more efficient and optimal manner.

Of course, without departing from the protective scope of the invention, the second comparison step can be executed in other equivalent ways which allow understanding which is the greatest production parameter in order to possibly modify the characteristics of the food in the operative step. For example, in accordance with an embodiment variant, in the second comparison step, a second difference is calculated between the objective production parameter PO and the actual production parameter PR. Therefore, in accordance with such embodiment variant, in the operative step, the characteristic of the food is modified if the second difference is greater than zero, while it is maintained unchanged if the second difference is smaller than or equal to zero.

In accordance with the preferred embodiment of the present invention, the objective production parameter PO is obtained by means of the following formula (IV): $PO = {γ}_{00} + {γ}_{11} \times SSOD + {γ}_{22} \times IOo$ in which:
IOo is an objective homogeneity index of the food, and
*γ*₀₀*, γ*₁₁ and *γ*₂₂ are third coefficients obtained by means of statistical regression,
SSOD is an objective quantity of dry matter ingested and digested by a bovine.

In particular, the objective quantity of dry matter ingested and digested by a bovine SSOD is calculated means of the following formula (V): $SSOD = QOSS \times DO$ in which:
QOSS is an objective quantity of ingested dry matter that is statistically obtained, and
DO is an objective digestibility parameter that is statically obtained.

In particular, the third coefficients are obtained by means of multivariate regression based on data obtained from a plurality of livestock farms. Advantageously, such third coefficients are contained in the mathematical model that implements the second calculation step.

Advantageously, in addition, the aforesaid homogeneity index IOo is statistically estimated, e.g. by means of the mathematical model, or calculated with methods of known type, e.g. by means of the method described in the patent IT 102016000047355, on an objective food quantity that is statistically determined. In such case, the homogeneity index is advantageously measured as a value comprised between 0 and 100.

Advantageously, the process preliminarily comprises a processing step, in which at least one mathematical model is processed, preferably so as to obtain the first coefficients, the second coefficients and/or the third coefficients of the above-reported formulas.

Advantageously, in addition, the mathematical model obtained in the processing step is used for obtaining the objective production parameter PO, the objective homogeneity index of the food IOo and/or the objective quantity of ingested dry matter QOSS.

More in detail, the processing step provides for identifying a plurality of livestock farms, i.e. of breeding facilities, and for detecting, for each of such livestock farms, a plurality of data points selected from among: a quantity of ingested dry matter QSS; an actual digestibility parameter DR, indicative of the digestibility of the quantity of ingested dry matter QSS; one or more composition data points DCi of the quantity of ingested dry matter QSS; an actual production parameter PR, indicative of the actual milk or meat production of the cattle; a homogeneity index IO of the food distributed to the animals.

In particular, the actual digestibility parameter DR can be determined by means of the formulas (IA), (IB) or (IC) that are reported above, or by means of experimental methods that are known in the art.

Advantageously, the processing step provides for determining, with statistical methods, such as for example multivariate analysis, the mathematical correlation between the actual digestibility parameter DR and the composition data points DCi.

Such correlation is comprised in the mathematical model and is for example represented by the following formula (VI) (analogous to the formula (I)): $DR = {β}_{0} + {\sum }_{n} \left(\pm {β}_{i}\times DCi\right)$

Consequently, advantageously, the processing step provides for determining, for example by means of multivariate regression, the first coefficients β₀ and βᵢ, and in particular β₀, β₁, β₂, β₃, β₄, β₅ and β₆, of the formulas (I), (I'), (I") and (VI). Therefore, advantageously, the potential digestibility parameter DP actually represents the digestibility that is expected with a specific composition of the food, based on the mathematical model that is statistically obtained on the mean of the analyzed livestock farms.

Advantageously, in addition, the processing step provides for determining a quantity of dry matter that is actually ingested and digested SSRD, e.g. based on the actual digestibility parameter DR and the quantity of ingested dry matter QSS by means of the following formula (VII) (analogous to the formula (III)): $SSRD = QSS \times DR$

Then, the processing step advantageously provides for determining, with statistical methods, such as for example multivariate analysis, the mathematical correlation between the quantity of dry matter actually ingested and digested SSRD (depending on the actual digestibility parameter DR) and the actual production parameter PR.

Such correlation is comprised in the mathematical model and is for example represented by the following formula (VIII) (analogous to the formula (II)): $PR = {γ}_{0} + {γ}_{1} \times SSPD + {γ}_{2} \times IO$

Consequently, advantageously the processing step provides for determining, for example by means of multivariate regression, the second coefficients *γ*₀, *γ*₁ and *γ*₂, of the formulas (II) and (VIII).

Therefore, advantageously, the potential production parameter PP actually represents the production that is expected with a specific composition of the food, based in the mathematical model that is statistically obtained on the mean of the analyzed livestock farms.

Preferably, the third coefficients *γ*₀₀*, γ*₁₁ and *γ*₂₂ are equal to the second coefficients *γ*₀, *γ*₁ and *γ*₂.

In accordance with a statistical analysis executed by the applicant, several possible values of the first coefficients are indicated in Table 1 and several possible values of the second coefficients are indicated Table 2, reported hereinbelow.

**Table 1 - Exemplifying values of the first coefficients**

| | *β₀* | *β₁* | *β₂* | *β₃* | *β₄* | *β₅* | *β₆* |
|---|---|---|---|---|---|---|---|
| *Value of β* | 0±120 | 0±20 | 0±10 | 0±5 | 0±30 | 0±35 | 0±17 |

**Table 2 - Exemplifying values of the second coefficients**

| | *γ*₀ | *γ*₁ | *γ*₂ |
|---|---|---|---|
| *Value of γ* | 0±120 | 0±10 | 0±8 |

Advantageously, the processing step also provides for selecting, from among the plurality of livestock farms, a group of optimal companies, in which the actual production parameter PR detected is greater than all the other livestock farms. Advantageously, the mean values of the data detected by such companies are taken as objective values, for example in the formulas (IV) and (V).

In accordance with the statistical analysis executed by the applicant, several mean values of the optimal digestibility parameters are indicated in Table 3 reported hereinbelow.

**Table 3 - Exemplifying values of the optimal digestibility parameters for several nutritional factors**

| *Nutritional factor* | *Optimal digestibility parameter (%) dairy cattle* | *Optimal digestibility parameter (%) beef cattle* |
|---|---|---|
| *Dry matter (SS)* | 61 | 63 |
| *Raw proteins (PG)* | 60 | 55 |
| *aNDF* | 38 | 43 |
| *Starch (CR)* | 98 | 96 |

In addition, it was indicated that for the best farms, on average the quantity of ingested dry matter is 25 kg for dairy cattle and 10 kg for beef cattle, in which the percentage of dry matter %SS in the distributed quantity of food is 50%. Advantageously, therefore, the aforesaid data can be used in the above-indicated formula (V) as objective values. Therefore, in this exemplifying case, the value of the objective quantity of ingested dry matter QOSS is 25 kg or 10 kg (respectively for dairy cattle and beef cattle) and the optimal digestibility value DO is respectively 61% or 63%.

Of course, without departing from the protective scope of the invention, the above-reported values can vary based on the considered livestock farms, on the zone where they are located and on the data collected in order to build the mathematical model.

Below, several non-limiting embodiments are reported in order to better understand how to possibly modify the operative characteristics or food characteristics following the first or second comparison step.

In accordance with a first embodiment of the process, in which the milk production in the breeding facility is considered, and one finds that the potential production parameter PP is greater than the actual production parameter PR, the lack of attainment of the potentially obtainable production is due to the operative characteristics. In particular, the lack of attainment of the potentially obtainable production could be due to the unsuitability of the mean lactation days of the dairy bovines, which is caused by the incorrect setting of the aforesaid operative characteristics that affect the wellbeing of the animal.

More in detail, it is known that the lactation curves of the bovines, i.e. the curve that represents the quantity of milk produced over time, initially has an increasing production phase up to reaching the maximum production of milk (known in the jargon as "lactation peak") at a period comprised between 60 and 90 days after delivery, before then progressively decreasing. Therefore, in order to be able to have high milk productions, it is advantageously necessary that the mean lactation days, i.e. the mean of the days in which the milk is collected, are close to the days necessary for reaching the lactation peak, compatibly with the duration of the pregnancy.

Therefore, it is known that it is important for bovines to once again be pregnant in the briefest possible time period after delivery. Such time comprised between a delivery and the subsequent conception is known with the name "delivery-conception period". Generally, the optimal reference values of the delivery-conception period are lower than 100 days, though sometimes some bovines may require longer lactation.

The present process also allows determining if it is necessary to modify the operative characteristics that are not correlated with the animal feeding in order to carry out operations suitable for decreasing the delivery-conception period.

In accordance with the first embodiment, a first modifiable operative characteristic, for decreasing the delivery-conception period, is the surface area available for each bovine. In particular, it is advantageous to increase the surface area available for each bovine so as to ensure a suitable animal wellbeing. More in detail, such first operative characteristic can be modified by means of the creation of a delivery box where the bovine can deliver isolated from the rest of the herd and for at least several hours per day take care of her calf.

As an alternative or in combination, such operative characteristic can be modified by means of the sizing of the post-delivery box such that the bovines that have delivered can access the manger without competition.

As an alternative or in combination, such operative characteristic can be further modified by means of the sizing of the bunks, for example bringing them to a surface area greater than 10 m²/bovine, preferably with about 16m²/bovine in the case of permanent litter.

In addition, advantageously, a second modifiable operative characteristic is the number of places occupied in manger. In particular, it is possible to decrease the number of places occupied in manger, bringing it to a number lower than 60%, preferably equal to about 50% of bovines with respect to the manger places. In this manner, competition is decreased, increasing the animal wellbeing.

In addition, advantageously, a third modifiable operative characteristic is the number of places occupied in a bunk. In particular, it is possible to decrease the number of places occupied in a bunk, bringing it to a number lower than 60%, preferably equal to about 50% of bovines with respect to the places in a bunk. In this manner, the bovines are allowed to rest on suitable spaces.

In addition, advantageously, a fourth modifiable operative characteristic is the extension of the manger or drinking trough front. In particular, it is possible to increase the extension of the drinking trough front, bringing it to an extension greater than 15 cm, preferably equal to about 20 cm of drinking trough for each bovine. In this manner, each bovine can have abundant drinking water available without competition.

In addition, advantageously, fifth modifiable operative characteristic is the frequency of the veterinary activities. In particular, it is possible to subject the bovines to veterinary visits in a systematic manner, in a period comprised between 7 and 15 days after delivery and repeat them advantageously on the animals at risk and/or suffering from pathologies. In this manner, a quick renewal of the sexual cycle is facilitated after delivery in the bovine by means of the control of uterine involution and of the onset of technopathies, such as ketosis, and of pathologies affecting the reproductive apparatus.

A further modifiable operative characteristic can be the improvement of the personnel for an optimal detection of the calories, possibly assisted by the use of specific movement sensors on the animals and the actuation of synchronization plans, and for the verification of the fertility of the semen and of the accuracy of the insemination operation.

In accordance with a second embodiment, in which one considers the milk production in the breeding facility and one finds that the objective production parameter PO is greater than the actual production parameter PR, the lack of attainment of the potentially obtainable production is due to the characteristics of the food, which therefore must be modified in order to increase the digestibility of the diet, i.e. the capacity of the animal to retain and absorb the ingested nutritional factors.

In accordance with the second embodiment, a first modifiable characteristic of the food for increasing the digestibility of the food is the formulation of the food recipe. In particular, the food recipe can be modified by controlling the quality of the food components, and in particular the presence of silage and anti-nutritional factors.

As an alternative or in combination, the formulation of the food recipe can be advantageously modified such that the chemical composition of the diet and in particular the ratio between proteins, non-structural carbohydrates and fiber are correct. For example, the speed of degradation of the different compounds can be evaluated so as to synchronize the availability of the nutrients useful for enhancing ruminant microbial activities.

As an alternative or in combination, in order to modify such first characteristic of the food, the supply of fiber can be varied in the food recipe, both in terms of cutting length and for structural quality. In particular, the use of suitable cutting lengths of the forage is important in order to ensure a suitable rumination and a speed of transit of the foods, in the digesting tube, capable of facilitating regular digestion times, without simultaneously reducing consumptions. In addition, it is advantageously important to reduce the cutting lengths in the presence of excessively structured fibers, i.e. if reduced rations between NDF and ADL are present.

In particular, if it is necessary to act on the length of the fiber, i.e. the cutting length, the ideal length can be calculated by means of the method described in the patent IT 102021000025703 or in the patent IT 102022000012275.

In addition, advantageously, a second modifiable characteristic of the food is the frequency with which the distribution step is executed. In particular, the frequency can be modified so as to control the regularity of the ingestion of the foods by the bovines, in particular verifying the number of daily meals and their duration.

In accordance with a third embodiment, in which one considers the production of meat in the breeding facility and one finds that the objective production parameter PO is greater than the actual production parameter PR, the lack of attainment of the potentially obtainable production is due to the characteristics of the food, which must therefore be modified in order to increase the digestibility of the diet, i.e. the capacity of the animal to retain and absorb the ingested nutritional factors. In accordance with the third embodiment, the modifiable characteristics of the food are those reported above for the second embodiment.

Advantageously, in addition, a third modifiable characteristic of the food is the selectionability of the food. In particular, in the event in which the cutting length of the rough forage is excessive, the cattle are able to select the food component and therefore ingest first the concentrates, strongly unbalancing the ruminant activities with strong risks of onset of acidosis. Consequently, in order to modify and in particular decrease the selectionability of the food, it is possible to decrease the cutting length of the forage, then controlling the cutting length and/or the selectionability, for example with the methods indicated above.

The general idea underlying the present invention, i.e. the idea to compare at least one potential production parameter PP and an actual beef or dairy production parameter PR in order to determine if it is necessary to modify operative characteristics, can also be applied to the production of methane or nitrogen, i.e. comparing a potential production parameter MP and an actual production parameter MR of methane or oxygen by the cattle of the breeding facility. Therefore, also forming the object of the invention is a process for controlling a cattle breeding facility analogous to that described above, but in which in place of the potential production PP, actual PR and advantageously beef and dairy objective PO parameters, potential MP, actual MR and advantageously objective MO production parameters of methane or nitrogen are calculated and compared.

The process according to the invention comprises a step of arranging a food, a step of setting, an analysis step and a distribution step provided with all the characteristics of the analogous steps described above.

The process also comprises a first calculation step, in which a potential digestibility parameter DP and a potential production parameter MP of methane or nitrogen are calculated.

In particular, the potential digestibility parameter DP, as above, is indicative of the digestibility of the food and is calculated as a function of the composition data points DCi.

In addition, the potential production parameter MP of methane or oxygen is indicative of an estimated quantity of production of methane or oxygen obtainable by the cattle, and calculated as a function of the potential digestibility parameter DP.

The process also comprises a detection step, in which an actual production parameter MR of methane or nitrogen is detected, indicative of the actual production of methane or nitrogen of the cattle, and a first comparison step, in which the comparison is executed between the potential production parameter MP and the actual production parameter MR of methane or nitrogen.

The process also comprises an operative step, in which if the potential production parameter MP is lower than the actual production parameter MR, the operative characteristic is modified.

Otherwise, if the potential production parameter MP of methane or nitrogen is greater than or equal to the actual production parameter MR, the operative characteristic is maintained unchanged.

Advantageously, in addition, the process comprises a second calculation step in which an objective production parameter MO is calculated, indicative of an objective production of methane or nitrogen.

Advantageously, in addition, the process comprises a second comparison step, in which the comparison is executed between the objective production parameter MO and the actual production parameter MR.

In particular, in the operative step, if the objective production parameter MO is lower than the actual production parameter MR of methane or nitrogen, at least one characteristic of the food is modified.

Otherwise, if the objective production parameter MO is greater than or equal to the actual production parameter MR, the characteristic of the food is maintained unchanged.

Advantageously, the potential production parameter MP and the optimal production parameter MO for methane or nitrogen can be calculated by means of the mathematical model and the above-indicated formulas, suitably adapted.

The invention thus described therefore attains the pre-established objects.

## Claims

1. Process for controlling a cattle breeding facility, which comprises:
- a step of arranging a food, in which at least one preset quantity of food is prepared for a dairy or beef cattle breeding facility;
- a setting step, in which at least one operative characteristic is set for said breeding facility in which said cattle are bred; wherein said at least one operative characteristic is selected from among: number of places occupied in a manger, extension of the manger or drinking trough front, number of places occupied in a bunk, surface area available for each bovine, presence of ventilation, presence of enzymes in the litter, sewage removal frequency, frequency of the veterinary activities, space available in the manger and/or in the stall for each bovine;
- an analysis step, in which said food is analyzed in order to obtain at least one composition data (DCi), comprising at least one percentage of dry matter (SS%) and/or one percentage of at least one nutritional factor in said food;
- a distribution step, in which said preset quantity of food is distributed along a manger to a plurality of cattle of said breeding facility;
said process being **characterized in that** it also comprises:
- a first calculation step, in which the following are calculated:
- a potential digestibility parameter (DP), which is indicative of the digestibility of said food and is calculated as a function of said at least one composition data (DCi);
wherein said potential digestibility parameter (DP) is calculated with the following formula (I): $DP = {β}_{0} + {\sum }_{n} \left(\pm {β}_{i}\times DCi\right)$ in which:
Dci is the value of one said composition data, and
β₀ and βᵢ are first coefficients obtained by means of statistical regression;
- a potential production parameter (PP), which is indicative of an estimated quantity of milk or meat production obtainable from said cattle, and calculated as a function of said potential digestibility parameter (DP);
wherein said potential production parameter (PP) is obtained by means of the following formula (II): $PP = {γ}_{0} + {γ}_{1} \times SSPD + {γ}_{2} \times IO$ in which:
IO is a food homogeneity index,
*γ*₀, *γ*₁ and *γ*₂ are second coefficients obtained by means of statistical regression, and
SSPD is a potential quantity of dry matter ingested and digested by a bovine and is calculated by means of the following formula (III): $SPPD = QSS \times DP$
in which:
QSS is a quantity of ingested dry matter, and
DP is the potential digestibility parameter;
- a detection step, in which an actual production parameter (PR) is detected, indicative of the actual milk or meat production of said cattle;
- a first comparison step, in which the comparison is executed between said potential production parameter (PP) and said actual production parameter (PR);
- an operative step, in which:
- if said potential production parameter (PP) is greater than said actual production parameter (PR), said at least one operative characteristic is modified;
- if said potential production parameter (PP) is less than or equal to said actual production parameter (PR), said at least operative characteristic is maintained unchanged.

2. Process according to claim 1, **characterized in that** in said analysis step said at least one nutritional factor of said at least one composition data (DCi) is selected from among: raw protein (PG) contained in the food, fibrous fraction (FF) contained in the food and reserve carbohydrates (CR) contained in the food.

3. Process according to claim 1 or 2, **characterized in that** said at least one composition data (DCi) also comprises at least one physical characteristic (CF) of said food.

4. Process according to any of the preceding claims, **characterized in that** in said formula (I), the following equation (I') holds: $\begin{matrix}{{\sum }_{n} \left(\pm {β}_{i}\times DCi\right) = {β}_{1} \times SS % \pm {β}_{2} \times PG % \pm β}_{3} \times LG % \pm {β}_{4} \times FF % \pm \\ {β}_{5} \times CR % \pm {β}_{6} \times CF\end{matrix}$ in which:
DCi is the value of one said composition data,
SS% is the percentage of dry matter contained in the food,
PG% is the percentage of raw protein contained in the food,
LG% is the percentage of raw lipids contained in the food,
FF% is the percentage of fibrous fraction contained in the food,
CR% is the percentage of reserve carbohydrates contained in the food,
CF is a physical characteristic of the food, and
β₀, βᵢ, β₁, β₂, β₃, β₄, β₅ and β₆ are first coefficients obtained by means of statistical regression.

5. Process according to any one of the preceding claims, **characterized in that** it comprises:
a second calculation step in which an objective production parameter (PO) is calculated, indicative of an objective production of milk or meat;
wherein said objective production parameter (PO) is obtained by means of the following formula (IV): $PO = {γ}_{00} + {γ}_{11} \times SSOD + {γ}_{22} \times IOo$
in which:
IOo is an index of objective homogeneity of the food, and
*γ*₀₀*, γ*₁₁ and *γ*₂₂ are third coefficients obtained by means of statistical regression,
SSOD is an objective quantity of dry matter ingested and digested by a bovine and
is calculated by means of the following formula (V): $SSOD = QOSS \times DO$
in which:
QOSS is an objective quantity of ingested dry matter that is statistically obtained, and
DO is an objective digestibility parameter that is statistically obtained;
a second comparison step, in which the comparison between said objective production parameter (PO) and said actual production parameter (PR) is executed;
in which in said operative step:
- if said objective production parameter (PO) is greater than said actual production parameter (PR), at least one characteristic of said food is modified;
- if said objective production parameter (PO) is smaller than or equal to said actual production parameter (PR), said at least one characteristic of said food is maintained unchanged;
wherein at least one characteristic of said food is selected from among: preset quantity of food, homogeneity of the food, selectability of the food, formulation of the recipe, frequency with which the distribution step is executed.

6. Process according to claim 5, **characterized in that** it preliminarily comprises a processing step, in which at least one mathematical model is processed so as to obtain said first coefficients, second coefficients and third coefficients,
wherein said first calculation step, in which said potential digestibility parameter (DP) and said potential production parameter (PP) are calculated, is implemented by means of said at least one mathematical model.

7. Process according to claim 5 and 6, **characterized in that** said mathematical model obtained in said processing step is used for obtaining said objective production parameter (PO).

8. Process according to claim 6 or 7, **characterized in that** said processing step provides for identifying a plurality of livestock farms, i.e. breeding facilities, and for detecting, for each of said livestock farms, a plurality of data selected from among: a quantity of ingested dry matter (QSS); an actual digestibility parameter (DR), indicative of the digestibility of the quantity of ingested dry matter (QSS); one or more composition data points (DCi) of the quantity of ingested dry matter (QSS); an actual production parameter (PR), indicative of the actual milk or meat production of the cattle; a homogeneity index (IO) of the food distributed to the animals;
wherein said processing step provides for determining with statistical methods the mathematical correlation between said actual digestibility parameter (DR) and said composition data points (DCi).

9. Process according to claim 8, **characterized in that** said statistical methods in said processing step provide for multivariate analysis.

10. Process according to claim 8 or 9, **characterized in that** said potential digestibility parameter (DP) represents the digestibility that is expected with a specific composition of the food, based on said mathematical model statistically obtained on the mean of said analyzed livestock farms;
and said potential production parameter (PP) represents the production that is expected with a specific composition of the food, based on said mathematical model statistically obtained on the mean of said analyzed livestock farms.

## Patentansprüche

1. Verfahren zur Steuerung einer Viehzuchtanlage, das Folgendes umfasst:
- einen Schritt zum Bereitstellen eines Futters, bei dem mindestens eine vorgegebene Menge an Futter für eine Milch- oder Rinderzuchtanlage vorbereitet wird;
- einen Einstellungsschritt, bei dem mindestens ein operativer Eigenschaft für die genannte Zuchtanlage festgelegt wird, in der die genannten Rinder gezüchtet werden; wobei der genannte mindestens eine operative Eigenschaft aus folgenden ausgewählt wird: Anzahl der belegten Plätze in einer Futterstation, Ausdehnung der Futterstation- oder Tränkefront, Anzahl der belegten Plätze in einer Liegebox, für jedes Rind verfügbare Fläche, Vorhandensein einer Belüftung, Vorhandensein von Enzymen in der Einstreu, Häufigkeit der Gülleentfernung, Häufigkeit der tierärztlichen Maßnahmen, für jedes Rind verfügbarer Platz in der Futterstation und/oder im Stand;
- einen Analyseschritt, bei dem das genannte Futter analysiert wird, um mindestens einen Zusammensetzungswert (DCi) zu ermitteln, der mindestens einen Trockenmasseanteil (SS¾) und/oder einen prozentualen Anteil mindestens eines Nährstofffaktors in dem genannten Futter umfasst;
- einen Verteilungsschritt, bei dem die genannte vorgegebene Futtermenge entlang einer Futterstation an eine Vielzahl von Rindern der genannten Zuchtanlage verteilt wird; wobei das genannte Verfahren **dadurch gekennzeichnet ist, dass** es außerdem Folgendes umfasst:
- einen ersten Berechnungsschritt, bei dem Folgendes berechnet wird:
- ein potenzieller Verdaulichkeitsparameter (DP), der Aufschluss über die Verdaulichkeit des genannten Futters gibt und abhängig von dem genannten mindestens einen Zusammensetzungswert (DCᵢ) berechnet wird; wobei der genannte potenzielle Verdaulichkeitsparameter (DP) anhand der folgenden Formel (I) berechnet wird: DP = β₀ + Σₙ(± βᵢ × DCᵢ) (I), wobei: DCᵢ der Wert eines genannten Zusammensetzungswerts ist und β₀ sowie βᵢ erste Koeffizienten sind, die mittels statistischer Regression ermittelt werden;
- ein potenzieller Produktionsparameter (PP), der Aufschluss über eine geschätzte Milch- oder Fleischerzeugungsmenge gibt, die aus den genannten Rindern erzielt werden kann, und der abhängig von dem genannten potenziellen Verdaulichkeitsparameter (DP) berechnet wird; wobei der genannte potenzielle Produktionsparameter (PP) anhand der folgenden Formel (II) ermittelt wird: PP = γ0 + γ₁ × SSPD + γ2 × IO (II), wobei: IO ein Homogenitätsindex des Futters ist, γ0, γ1 und γ2 zweite Koeffizienten sind, die mittels statistischer Regression ermittelt werden, und SSPD eine potenzielle Menge von von einem Rind aufgenommener und verdauter Trockenmasse ist, die anhand der folgenden Formel (III) berechnet wird: SSPD = QSS × DP (III), wobei: QSS die aufgenommene Trockenmasse und DP der potenzielle Verdaulichkeitsparameter ist;
- einen Erfassungsschritt, bei dem ein tatsächlicher Produktionsparameter (PR) ermittelt wird, der Aufschluss über die tatsächliche Milch- oder Fleischproduktion der genannten Rinder gibt;
- einen ersten Vergleichsschritt, bei dem der Vergleich zwischen dem genannten potenziellen Produktionsparameter (PP) und dem tatsächlichen Produktionsparameter (PR) durchgeführt wird;
- einen Verfahrensschritt, bei dem:
- wenn der genannte potenzielle Produktionsparameter (PP) größer ist als der genannte tatsächliche Produktionsparameter (PR), der genannte mindestens eine operative Eigenschaft geändert wird;
- wenn der genannte potenzielle Produktionsparameter (PP) kleiner oder gleich dem genannten tatsächlichen Produktionsparameter (PR) ist, der genannte mindestens eine operative Eigenschaft unverändert bleibt.

2. Verfahren nach Anspruch 1, **dadurch gekennzeichnet, dass** bei dem genannten Analyseschritt der genannte mindestens einen Nährstofffaktor des genannten mindestens einen Zusammensetzungswerts (DCᵢ) unter Folgendem ausgewählt wird: im Futter enthaltenem Rohprotein (PG), im Futter enthaltener Ballaststofffraktion (FF) und im Futter enthaltenen Reservekohlenhydraten (CR).

3. Verfahren nach Anspruch 1 oder 2, **dadurch gekennzeichnet, dass** der genannte mindestens eine Zusammensetzungswert (DCi) außerdem mindestens eine physikalische Eigenschaft (CF) des genannten Futters umfasst.

4. Verfahren gemäß einem beliebigen der vorangegangenen Ansprüche, **dadurch gekennzeichnet, dass** bei der genannten Formel (I) die folgende Gleichung (I') gilt: Σₙ(± βᵢ × DCᵢ) = β₁ × SS% ± β₂ × PG% ± β₃ × LG% ± β₄ × FF% ± β₅ × CR% ± β₆ × CF (I'), wobei: DCᵢ der Wert eines genannten Zusammensetzungswerts ist, SS% der prozentuale Anteil an im Futter enthaltener Trockenmasse ist, PG% der prozentuale Anteil an im Futter enthaltenem Rohprotein ist, LG% der prozentuale Anteil an im Futter enthaltenem Rohfett ist, FF% der prozentuale Anteil der im Futter enthaltenen Ballaststoffe ist, CR% der prozentuale Anteil der im Futter enthaltenen Reservekohlenhydrate ist, CF eine physikalische Eigenschaft des Futters ist und β₀, βᵢ, β₁, β₂, β₃, β₄, β₅ und β₆ erste Koeffizienten sind, die mittels statistischer Regression ermittelt werden.

5. Verfahren nach einem beliebigen der vorangegangenen Ansprüche, **dadurch gekennzeichnet, dass** es Folgendes umfasst: einen zweiten Berechnungsschritt, bei dem ein objektiver Produktionsparameter (PO) berechnet wird, der Aufschluss über eine objektive Produktion von Milch oder Fleisch gibt; wobei der genannte objektive Produktionsparameter (PO) anhand der folgenden Formel (IV) ermittelt wird: PO = γ₀₀ + γ₁₁ × SSOD + γ₂₂ × IOo (IV), wobei: IOo ein Index für die objektive Homogenität des Futters ist und γ₀₀, γ₁₁ und γ₂₂ dritte Koeffizienten sind, die mittels statistischer Regression ermittelt werden, SSOD eine objektive Menge von von einem Rind aufgenommener und verdauter Trockenmasse ist und anhand der folgenden Formel (V) berechnet wird: SSOD = QOSS × DO (V), wobei: QOSS eine statistisch ermittelte objektive Menge an aufgenommener Trockenmasse ist und DO ein statistisch ermittelter objektiver Verdaulichkeitsparameter ist; ein zweiter Vergleichsschritt, bei dem der Vergleich zwischen dem genannten objektiven Produktionsparameter (PO) und dem genannten tatsächlichen Produktionsparameter (PR) durchgeführt wird; wobei bei dem genannten operativen Schritt:
- wenn der genannte objektive Produktionsparameter (PO) größer ist als der genannte tatsächliche Produktionsparameter (PR) ist, mindestens eine Eigenschaft des genannten Futters verändert wird;
- wenn der genannte objektive Produktionsparameter (PO) kleiner oder gleich dem genannten tatsächlichen Produktionsparameter (PR) ist, die genannte mindestens eine Eigenschaft des genannten Futters unverändert bleibt; wobei die mindestens eine Eigenschaft des genannten Futters unter Folgendem ausgewählt wird: vorgegebene Futtermenge, Homogenität des Futters, Auswählbarkeit des Futters, Rezeptur, Häufigkeit, mit der der Verteilungsschritt durchgeführt wird.

6. Verfahren nach Anspruch 5, **dadurch gekennzeichnet, dass** es vorab einen Verarbeitungsschritt umfasst, bei dem mindestens ein mathematisches Modell verarbeitet wird, um die genannten ersten Koeffizienten, zweite Koeffizienten und dritte Koeffizienten zu ermitteln, wobei der genannte erste Berechnungsschritt, bei dem der genannte potenzielle Verdaulichkeitsparameter (DP) und der genannte potenzielle Produktionsparameter (PP) berechnet werden, mittels des genannten mindestens einen mathematischen Modells implementiert wird.

7. Verfahren nach Anspruch 5 und 6, **dadurch gekennzeichnet, dass** das bei dem genannten Verarbeitungsschritt ermittelte mathematische Modell zur Ermittlung des genannten objektiven Produktionsparameters (PO) verwendet wird.

8. Verfahren nach Anspruch 6 oder 7, **dadurch gekennzeichnet, dass** der genannte Verarbeitungsschritt die Identifizierung einer Vielzahl von Tierhaltungsbetrieben, d. h. Zuchtbetrieben vorsieht sowie die Erfassung einer Vielzahl von Daten für jeden der genannten Tierhaltungsbetriebe, die unter Folgenden gewählt werden: einer Menge ausgenommener Trockenmasse (QSS); einem tatsächlichen Verdaulichkeitsparameter (DR), der Aufschluss über die Verdaulichkeit der Menge aufgenommener Trockenmasse (QSS) gibt; einen oder mehrere Zusammensetzungsdatenpunkte (DCᵢ) der Menge aufgenommener Trockenmasse (QSS); einen tatsächlichen Produktionsparameter (PR), der Aufschluss über die tatsächliche Milch- oder Fleischproduktion der Rinder gibt; einen Homogenitätsindex (IO) des an die Tiere verfütterten Futters; wobei der genannte Verarbeitungsschritt die Bestimmung der mathematischen Korrelation zwischen dem genannten tatsächlichen Verdaulichkeitsparameter (DR) und den genannten Zusammensetzungsdatenpunkten (DCᵢ) mittels statistischer Verfahren vorsieht.

9. Verfahren nach Anspruch 8, **dadurch gekennzeichnet, dass** die genannten statistischen Verfahren bei dem genannten Verarbeitungsschritt eine multivariate Analyse vorsehen.

10. Verfahren nach Anspruch 8 oder 9, **dadurch gekennzeichnet, dass** der genannte potenzielle Verdaulichkeitsparameter (DP) die Verdaulichkeit darstellt, die bei einer spezifischen Zusammensetzung des Futtermittels zu erwarten ist, basierend auf dem genannten mathematischen Modell, das statistisch aus dem Mittelwert der genannten analysierten Tierhaltungsbetriebe ermittelt wird; und der genannte potenzielle Produktionsparameter (PP) die Produktion darstellt, die bei einer spezifischen Zusammensetzung des Futtermittels erwartet wird, basierend auf dem genannten mathematischen Modell, das statistisch aus dem Mittelwert der genannten analysierten Tierhaltungsbetriebe ermittelt wurde.

## Revendications

1. Procédé de commande d'une installation d'élevage de bétail comprenant :
- une étape de préparation d'une ration alimentaire, au cours de laquelle au moins une quantité prédéfinie de ration alimentaire est préparée pour une installation d'élevage de bétail laitier ou à viande ;
- une étape de configuration, au cours de laquelle au moins une caractéristique opérationnelle est définie pour ladite installation d'élevage dans laquelle ledit bétail est élevé ; ladite au moins une caractéristique opérationnelle étant choisie parmi : le nombre de places occupées dans une mangeoire, la longueur de la façade de la mangeoire ou de l'abreuvoir, le nombre de places occupées dans une auge, la surface disponible pour chaque bovin, la présence d'une ventilation, la présence d'enzymes dans la litière, la fréquence d'évacuation du lisier, la fréquence des interventions vétérinaires, l'espace disponible dans la mangeoire et/ou dans le box pour chaque bovin ;
- une étape d'analyse, au cours de laquelle ladite ration alimentaire est analysée afin d'obtenir au moins une donnée de composition (DCi), comprenant au moins un pourcentage de matière sèche (SS¾) et/ou un pourcentage d'au moins un facteur nutritionnel présent dans ladite ration alimentaire ;
- une étape de distribution, au cours de laquelle ladite quantité prédéfinie de ration alimentaire est distribuée le long d'une mangeoire à une pluralité bétail de ladite installation d'élevage ;
ledit procédé étant **caractérisé en ce qu'**il comprend en outre :
- une première étape de calcul, au cours de laquelle sont calculés les éléments suivants :
- un paramètre de digestibilité potentielle (DP), qui est représentatif de la digestibilité de ladite ration alimentaire et qui est calculé en fonction de ladite au moins une donnée de composition (DCᵢ) ; ledit paramètre de digestibilité potentielle (DP) étant calculé à l'aide de la formule (I) suivante : DP = β₀ + Σₙ(± βᵢ × DCᵢ) (I) où : DCᵢ est la valeur d'une desdites données de composition, et β₀ et βᵢ sont les premiers coefficients obtenus par régression statistique ;
- un paramètre de production potentielle (PP), qui est représentatif d'une quantité estimée de production de lait ou de viande pouvant être obtenue à partir dudit bétail, et qui est calculé en fonction dudit paramètre de digestibilité potentielle (DP) ; ledit paramètre de production potentielle (PP) étant obtenu à l'aide de la formule (II) suivante : PP = γ0 + γ1 × SSPD + γ2 × IO (II) où : IO est un indice d'homogénéité de la ration alimentaire, γ0, γ1 et γ2 sont des deuxièmes coefficients obtenus par régression statistique, et SSPD est une quantité potentielle de matière sèche ingérée et digérée par un bovin, calculée à l'aide de la formule (III) suivante : SSPD = QSS × DP (III) où : QSS est une quantité de matière sèche ingérée et DP est le paramètre de digestibilité potentielle ;
- une étape de détection, au cours de laquelle un paramètre de production réelle (PR) est détecté, qui est représentatif de la production réelle de lait ou de viande dudit bétail ;
- une première étape de comparaison, au cours de laquelle la comparaison est effectuée entre ledit paramètre de production potentielle (PP) et ledit paramètre de production réelle (PR) ;
- une étape opérationnelle, au cours de laquelle :
- si ledit paramètre de production potentielle (PP) est supérieur audit paramètre de production réelle (PR), ladite au moins une caractéristique opérationnelle est modifiée ;
- si ledit paramètre de production potentielle (PP) est inférieur ou égal audit paramètre de production réelle (PR), ladite au moins une caractéristique opérationnelle reste inchangée.

2. Procédé selon la revendication 1, **caractérisé en ce que**, dans ladite étape d'analyse, ledit au moins un facteur nutritionnel de ladite au moins une donnée de composition (DCᵢ) est choisi parmi : les protéines brutes (PG) contenues dans la ration alimentaire, la fraction fibreuse (FF) contenue dans la ration alimentaire et les glucides de réserve (CR) contenus dans la ration alimentaire.

3. Procédé selon la revendication 1 ou 2, **caractérisé en ce que** ladite au moins une donnée de composition (DCi) comprend également au moins une caractéristique physique (CF) de ladite ration alimentaire.

4. Procédé selon l'une quelconque des revendications précédentes, **caractérisé en ce que**, dans ladite formule (I), l'équation (I') suivante s'applique : Σₙ(± βᵢ × DCᵢ) = β₁ × SS% ± β₂ × PG% ± β₃ × LG% ± β₄ × FF% ± β₅ × CR% ± β₆ × CF (I') où : DCᵢ est la valeur d'une desdites données de composition, SS% est le pourcentage de matière sèche contenue dans la ration alimentaire, PG% est le pourcentage de protéines brutes contenues dans la ration alimentaire, LG% est le pourcentage de lipides bruts contenus dans la ration alimentaire, FF% est le pourcentage de fraction fibreuse contenue dans la ration alimentaire, CR% est le pourcentage de glucides de réserve contenus dans la ration alimentaire, CF est une caractéristique physique de la ration alimentaire, et β₀, βᵢ, β₁, β₂, β₃, β₄, β₅ et β₆ sont les premiers coefficients obtenus par régression statistique.

5. Procédé selon l'une quelconque des revendications précédentes, **caractérisé en ce qu'**il comprend : une deuxième étape de calcul au cours de laquelle un paramètre de production objective (PO) est calculé, qui est représentatif d'une production objective de lait ou de viande ; ledit paramètre de production objective (PO) étant obtenu à l'aide de la formule (IV) suivante : PO = γ₀₀ + γ₁₁ × SSOD + γ₂₂ × IOo (IV) où : IOo est un indice d'homogénéité objective de la ration alimentaire, γ₀₀, γ₁₁ et γ₂₂ sont des troisièmes coefficients obtenus par régression statistique, et SSOD est une quantité objective de matière sèche ingérée et digérée par un bovin, calculée à l'aide de la formule suivante (V) : SSOD = QOSS × DO (V) où : QOSS est une quantité objective de matière sèche ingérée, obtenue par des méthodes statistiques, et DO est un paramètre digestibilité objective obtenu par des méthodes statistiques ; une deuxième étape de comparaison, au cours de laquelle est effectuée la comparaison entre ledit paramètre de production objective (PO) et ledit paramètre de production réelle (PR) ; dans lequel, au cours de ladite étape opérationnelle :
- si ledit paramètre de production objective (PO) est supérieur audit paramètre de production réelle (PR), au moins une caractéristique de ladite ration alimentaire est modifiée ;
- si ledit paramètre de production objective (PO) est inférieur ou égal audit paramètre de production réelle (PR), ladite au moins une caractéristique de ladite ration alimentaire reste inchangée ; ladite au moins une caractéristique de ladite ration alimentaire étant choisie parmi : la quantité prédéfinie de ration alimentaire, l'homogénéité de la ration alimentaire, la sélectivité de la ration alimentaire, la formulation de la recette et la fréquence à laquelle l'étape de distribution est exécutée.

6. Procédé selon la revendication 5, **caractérisé en ce qu'**il comprend au préalable une étape de traitement, au cours de laquelle au moins un modèle mathématique est traité de manière à obtenir lesdits premiers coefficients, deuxièmes coefficients et troisièmes coefficients, dans lequel ladite première étape de calcul, au cours de laquelle ledit paramètre de digestibilité potentielle (DP) et ledit paramètre de production potentielle (PP) sont calculés, est mise en œuvre au moyen dudit au moins un modèle mathématique.

7. Procédé selon les revendications 5 et 6, **caractérisé en ce que** ledit modèle mathématique obtenu au cours de ladite étape de traitement est utilisé pour déterminer ledit paramètre de production objective (PO).

8. Procédé selon la revendication 6 ou 7, **caractérisé en ce que** ladite étape de traitement consiste à identifier une pluralité d'exploitations d'élevage, c'est-à-dire des installations d'élevage, et à détecter, pour chacune desdites exploitations d'élevage, une pluralité de données choisies parmi : une quantité de matière sèche ingérée (QSS) ; un paramètre de digestibilité réelle (DR), représentatif de la digestibilité de la quantité de matière sèche ingérée (QSS) ; un ou plusieurs points de données de composition (DCᵢ) de la quantité de matière sèche ingérée (QSS) ; un paramètre de production réelle (PR), représentatif de la production réelle de lait ou de viande du bétail ; un indice d'homogénéité (IO) de la ration alimentaire distribuée aux animaux ; ladite étape de traitement consistant à déterminer, à l'aide de méthodes statistiques, la corrélation mathématique entre ledit paramètre de digestibilité réelle (DR) et lesdits points de données de composition (DCᵢ).

9. Procédé selon la revendication 8, **caractérisé en ce que** lesdites méthodes statistiques utilisées dans ladite étape de traitement prévoient une analyse multivariée.

10. Procédé selon la revendication 8 ou 9, **caractérisé en ce que** ledit paramètre de digestibilité potentielle (DP) représente la digestibilité attendue pour une composition spécifique de la ration alimentaire, sur la base dudit modèle mathématique obtenu statistiquement sur la moyenne desdites exploitations d'élevage analysées ; et ledit paramètre de production potentielle (PP) représente la production attendue pour une composition spécifique de la ration alimentaire, sur la base dudit modèle mathématique obtenu statistiquement sur la moyenne desdites exploitations d'élevage analysées.
